# EUROPÄISCHE PATENTANMELDUNG

(11) **EP 2 556 823 A2**
(43) Veröffentlichungstag der Anmeldung: **13.02.2013**
(21) Anmeldenummer: 12178857.4
(22) Anmeldetag: 01.08.2012
(51) Int. Cl.: A61K 8/99, A61Q 19/10

(54) **Hautbarrierestärkendes Reinigungsmittel enthaltend ein auf probiotischen Mikroorganismen-basierendes Ferment**

(30) Priorität: 10.08.2011 DE 102011080715
(71) Anmelder: Henkel AG & Co. KGaA, 40589 Düsseldorf (DE)
(72) Erfinder: Heide, Barbara, 47809 Krefeld (DE); Schmitz, Stefanie, 41189 Mönchengladbach (DE)

(57) **Zusammenfassung**

Die Erfindung betrifft kosmetische Reinigungsmittel, die in einem geeigneten Träger
a) mindestens ein Alkylsulfat- und/oder ein Alkylpolyglykolethersulfatsalz,
b) mindestens ein amphoteres und/oder zwitterionisches Tensid und
c) mindestens ein auf probiotischen Mikroorganismen-basierendes Ferment enthalten, wobei die Mikroorganismen aus Milch isoliert wurden.

Die Reinigungsmittel sind besonders mild. Sie stärken die natürliche Hautbarriere und eignen sich für die Reinigung besonders sensibler Hautpartien wie der Intimregion.

## Beschreibung

Die Erfindung liegt auf dem Gebiet der Kosmetik und betrifft Reinigungsmittel, die eine Tensidmischung sowie ein spezielles Ferment enthalten.

Kosmetische Reinigungsmittel für die Haut und/oder die Haare wie flüssige Seifen, Shampoos, Duschbäder, Schaumbäder sowie Dusch- und Waschgele sind lange bekannt und werden fortwährend den sich ändernden Bedürfnissen der Verbraucher angepasst.

So müssen moderne Hautreinigungsmittel neben der Reinigung eine Reihe von Erfordernissen erfüllen, die als Sekundärerfordernisse bezeichnet werden.

Verbesserung der Haut- und/oder Schleimhautverträglichkeit, Verringerung der Austrocknung der Haut, Verbesserung des Feuchtigkeitsgehalts der Haut und/oder Vermeidung oder Verminderung von Hautbarriereschädigungen sind typische Sekundärerfordernisse, die ein Hautreinigungsmittel heutzutage erfüllen muss.

Gesunde Haut ist dicht besiedelt mit Mikroorganismen (beispielsweise Bakterien und Pilzen), die einen natürlichen Bestandteil der Hautoberfläche darstellen und als Haut(mikro)flora bezeichnet werden. Intakte Haut wird durch die Mikroorganismen nicht beeinträchtigt, denn sie verfügt über eine natürliche Barrierefunktion, die das Eindringen der Keime in die Haut verhindert.

Darüber hinaus schützt die natürliche Hautflora die Haut vor dem Eindringen fremder Keime. Erhöhte Feuchtigkeit auf der Haut führt zu einer erhöhten Keimdichte, so dass Hautregionen wie beispielsweise die Intimregion, die Achselhöhlen und/oder die Zehenzwischenräume besonders schnell mit Krankheitserregern befallen werden können, sobald die Hautmikroflora und/oder die Hautbarriere aus ihrem natürlichen Gleichgewicht gebracht werden.

Als "Hautbarriere" bezeichnet man die äußerste Schicht der Epidermis (Hornschicht, Stratum corneum), die für den natürlichen Schutz der Haut vor Umwelteinflüssen und/oder Austrocknung verantwortlich ist. Aufgrund des Kontaktes der Hornschicht mit der Umwelt wird diese fortwährend abgenutzt und muss kontinuierlich erneuert werden.

Eine wichtige Funktion der Hornschicht ist die Beeinflussung des Wasserbindevermögens und der Wasserdurchlässigkeit der Haut. Sie regelt den Wassernachschub und sorgt durch ihren Gehalt an Lipiden dafür, dass auch unter ungünstigen Bedingungen eine völlige Austrocknung der Haut üblicherweise vermieden wird.

Aufgrund von Umwelteinflüssen, Behandlungen und/oder Verletzungen der Haut kann der Zustand eintreten, dass sich die Hornschicht nicht oder nicht schnell genug erneuert, wodurch sie ihre Elastizität verliert und spröde oder brüchig werden kann.

Sprödigkeit und/oder Brüchigkeit der Hornschicht erleichtern das Eindringen schädlicher Keime. Die nachträgliche Wiederherstellung der Elastizität der Hornschicht durch äußere Zuführung von Lipiden ist dann nicht mehr ausreichend.

Ein wichtiger Aspekt bei der Herstellung fortschrittlicher Hautreinigungsmittel ist daher die Suche nach Wirkstoffen, die die Hautbarriere nachhaltig stärken und die sich problemlos in kosmetische Reinigungsformulierungen einarbeiten lassen.

Zu beachten ist, dass bestimmte Tenside, die üblicherweise in kosmetischen Reinigungsmitteln eingesetzt werden (müssen), ebenfalls eine Schädigung der Hautbarriere hervorrufen können.

Es muss demnach die Balance zwischen Reinigung und Schutz der Haut gefunden werden.

Der vorliegenden Erfindung lag die Aufgabe zugrunde, besonders milde kosmetische Reinigungsmittel herzustellen, die gut haut- und schleimhautverträglich sind.

Weiterhin sollten die Reinigungsmittel zum Schutz der Mikroflora der menschlichen Haut und/oder zur Stabilisierung der Hautbarriere gegen schädigende Umwelteinflüsse geeignet sein. Insbesondere sollten kosmetische Reinigungsmittel entwickelt werden, die die zuvor genannten Anforderungen auch bei niedrigen pH-Werten erfüllen, und die auch für die Reinigung besonders sensibler Hautpartien - wie beispielsweise der Intimregion - geeignet sind.

Gegenstand der Erfindung ist ein kosmetisches Reinigungsmittel, das in einem geeigneten Träger
a) mindestens ein Alkylsulfat- und/oder ein Alkylpolyglykolethersulfatsalz,
b) mindestens ein amphoteres und/oder zwitterionisches Tensid und
c) mindestens ein auf probiotischen Mikroorganismen-basierendes Ferment enthält, wobei die Mikroorganismen aus Milch isoliert wurden.

Unter einem geeigneten Träger wird bevorzugt ein wässriger oder wässrig-alkoholischer Träger verstanden.

Bevorzugt enthält der kosmetische Träger mindestens 50 Gew.-%, mehr bevorzugt mindestens 60 Gew.-% und besonders bevorzugt mindestens 70 Gew.-% Wasser.

Weiterhin kann der kosmetische Träger 0,01 bis 40 Gew.-%, bevorzugt 0,05 bis 35 Gew.-% und insbesondere 0,1 bis 30 Gew.-% mindestens eines Alkohols enthalten, der ausgewählt sein kann aus Ethanol, Ethyldiglykol, 1-Propanol, 2-Propanol, Isopropanol, 1,2-Propylenglycol, Glycerin, Diglycerin, Triglycerin, 1-Butanol, 2-Butanol, 1,2-Butandiol, 1,3-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Polyethylenglycole, Sorbitol, Sorbitan, Benzylalkohol, Phenoxyethanol oder Mischungen dieser Alkohole.

Bevorzugt sind die wasserlöslichen Alkohole.

Besonders bevorzugt sind Ethanol, Ethyldiglykol, 1-Propanol, 2-Propanol, Isopropanol, 1,2-Propylenglycol, Glycerin, Benzylalkohol und/oder Phenoxyethanol sowie Mischungen dieser Alkohole.

Zur Erzielung optimaler Milde und Pflege enthalten erfindungsgemäße Reinigungsmittel das (die) Alkylsulfat- und/oder Alkylpolyglykolethersulfatsalz(e) a) und das (die) amphotere(n) Tensid(e) b) bevorzugt in einem Gewichtsverhältnis a) : b) im Bereich von 1 : 2 bis 10 : 1, besonders bevorzugt im Bereich von 1 : 1 bis 8 : 1 und insbesondere im Bereich von 2 : 1 bis 5 : 1.

Geeignete Alkylsulfat- und/oder Alkylpolyglykolethersulfatsalze a), die in den erfindungsgemäßen Reinigungsmitteln eingesetzt werden können, entsprechen bevorzugt der Formel R-O(CH₂-CH₂O)ₓ-OSO₃⁻ X⁺, in der R bevorzugt für eine lineare oder verzweigte, gesättigte oder ungesättigte Alkylgruppe mit 8 bis 30 C-Atomen, x für 0 oder eine Zahl von 1 bis 12 und X für ein Alkali-, ein Erdalkali-, ein Ammonium- oder ein Alkanolaminion steht.

Besonders bevorzugt aufgrund ihrer sehr guten Schäumeigenschaften und Milde sind geradkettige oder verzweigte Alkylethersulfate, die einen Alkylrest mit 8 bis 18 und insbesondere mit 10 bis 16 C-Atomen, sowie 1 bis 6 und insbesondere 2 bis 4 Ethylenoxideinheiten enthalten.

Insbesondere bevorzugt sind die Natrium-, Magnesium und/oder Triethanolaminsalze linearer oder verzweigter Lauryl-, Tridecyl- und/oder Myristylsulfate, die einen mittleren Ethoxylierungsgrad von 2 bis 4 aufweisen.

Das oder die Alkylsulfat- und/oder Alkylpolyglykolethersulfatsalz(e) a) wird (werden) in den erfindungsgemäßen Reinigungsmitteln bevorzugt in Mengen von 0,1 bis 15 Gew.-%, mehr bevorzugt von 0,25 bis 12,5 Gew.-%, besonders bevorzugt von 0,5 bis 10 Gew.-% und insbesondere von 1 bis 8 Gew.-% eingesetzt, wobei sich die Mengenangaben auf das Gesamtgewicht des Reinigungsmittels beziehen.

Geeignete amphotere und/oder zwitterionische Tenside b) wird (werden) in den erfindungsgemäßen Reinigungsmitteln bevorzugt in Mengen von 0,05 bis 10 Gew.-%, mehr bevorzugt von 0,1 bis 8 Gew.-%, besonders bevorzugt von 0,25 bis 6,5 Gew.-% und insbesondere von 0,5 bis 5 Gew.-% eingesetzt, wobei sich die Mengenangaben auf das Gesamtgewicht des Reinigungsmittels beziehen.

Geeignete amphotere und/oder zwitterionische Tenside b) können bevorzugt ausgewählt sein aus einer oder mehreren Verbindungen der nachfolgenden Formeln (I) bis (VII), in denen der Rest R für einen geradkettigen oder verzweigten, gesättigten oder ein- oder mehrfach ungesättigten Alkyl- oder Alkenylrest mit 7 bis 23 Kohlenstoffatomen (Formeln (I) und (II)) oder für einen geradkettigen oder verzweigten, gesättigten oder ein- oder mehrfach ungesättigten Alkyl- oder Alkenylrest mit 8 bis 24 Kohlenstoffatomen (Formeln (III) bis (VII)) steht

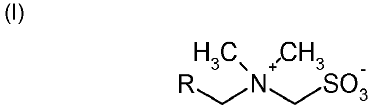

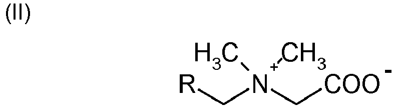

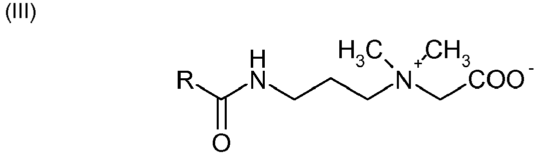

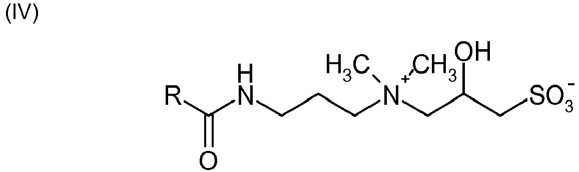

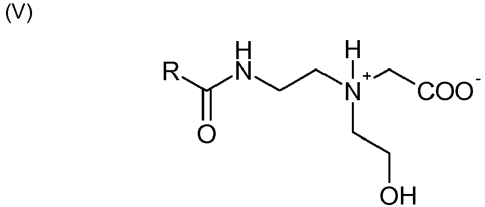

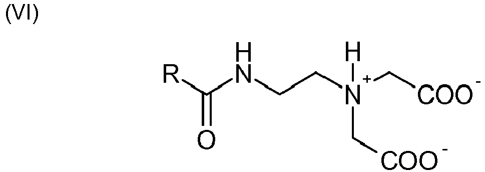

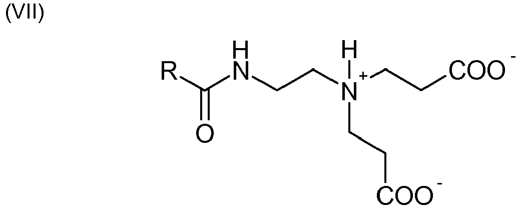

Bevorzugte amphotere und/oder zwitterionische Tenside einer der zuvor genannten Formeln (I) bis (VII) enthalten als Rest R überwiegend einen geradkettigen oder verzweigten, gesättigten, ein- oder mehrfach ungesättigten Alkylrest mit 8 bis 20, mehr bevorzugt von 8 bis 16 und insbesondere mit 8 bis 12 C-Atomen.

Mehr bevorzugt sind amphotere und/oder zwitterionische Tenside, bei denen sich der Rest R von Kokosfett ableitet.

Besonders bevorzugt sind amphotere und/oder zwitterionische Tenside der Formeln (III), (V), (VI) und (VII).

Insbesondere bevorzugt sind die unter den INCI-Bezeichnungen Cocamidopropylbetain und/oder Cocoamphodiacetate bekannten und im Handel von mehreren Anbietern erhältlichen amphoteren Tenside.

Unter "probiotischen Mikroorganismen" im Sinne der Erfindung werden lebende Mikroorganismen verstanden, die in einer adäquaten Menge einen positiven Effekt auf der Haut hervorrufen können. Unter einem positiven Effekt werden insbesondere die Stärkung der natürlichen Hautflora und/oder die Stärkung der natürlichen Hautbarriere verstanden.

Die für die Verwendung in den erfindungsgemäßen Reinigungsmitteln geeigneten probiotischen Mikroorganismen werden bevorzugt aus grampositiven, anaeroben Milchsäurebakterien (Lactobacillales) isoliert, die sich dadurch auszeichnen, dass sie bei der Fermentation von Zucker Milchsäure produzieren.

Entsprechend ihrer Morphologie können die Milchsäurebakterien generell in zwei Gattungen unterteilt werden; die Gattung *Lactobacillus* und die Gattung *Lactococcus.*

Geeignete Milchsäurebakterien der Gattung *Lactobacillus* können bevorzugt ausgewählt sein aus: *Lactobacillus acidophilus, amylovorus, casei, rhamnosus, brevis, crispatus, delbrueckii (*subsp. *bulgaricus, lactis), fermentum, johnsonii, helveticus, paracasei, plantarum, salivarius* und/oder *alimentarius.*

Geeignete Milchsäurebakterien der Gattung *Lactococcus* können bevorzugt ausgewählt sein aus: *Lactococcus lactis* (subsp. *lactis, cremoris* und/oder *hordniae*)*, Lactococcus garvieae, Lactococcus plantarum, Lactococcus raffinolactis* und/oder *Lactococcus piscium.*

Besonders bevorzugt für die Verwendung in den erfindungsgemäßen Reinigungsmitteln ist beispielsweise ein Ferment, das unter der INCI-Bezeichnung "Lactococcus Ferment and Propylene Glycol" und unter der Handelsbezeichnung "Technobion^{®} BL LS 8407" von der Firma Cognis im Handel erhältlich ist.

Geeignete Fermente können in den erfindungsgemäßen Reinigungsmitteln - bezogen auf deren Gesamtgewicht - bevorzugt in Mengen von 0,01 bis 10 Gew.-%, mehr bevorzugt von 0,05 bis 7,5 Gew.-%, besonders bevorzugt von 0,1 bis 5 Gew.-% und insbesondere von 0,5 bis 3 Gew.-% eingesetzt werden.

In einer ersten bevorzugten Ausführungsform enthalten erfindungsgemäße Reinigungsmittel - bezogen auf deren Gesamtgewicht -
- 0,1 bis 15 Gew.-% mindestens eines geradkettigen oder verzweigten Alkylethersulfats, das einen Alkylrest mit 8 bis 18 und insbesondere mit 10 bis 16 C-Atomen, sowie 1 bis 6 und insbesondere 2 bis 4 Ethylenoxideinheiten aufweist,
- 0,05 bis 10 Gew.-% mindestens eines amphoteren und/oder zwitterionischen Tensids b), das ausgewählt sein kann aus einer oder mehreren Verbindungen der nachfolgenden Formeln (I) bis (VII), in denen der Rest R für einen geradkettigen oder verzweigten, gesättigten oder ein- oder mehrfach ungesättigten Alkyl- oder Alkenylrest mit 7 bis 23 Kohlenstoffatomen (Formeln (I) und (II)) oder für einen geradkettigen oder verzweigten, gesättigten oder ein- oder mehrfach ungesättigten Alkyl- oder Alkenylrest mit 8 bis 24 Kohlenstoffatomen (Formeln (III) bis (VII)) steht 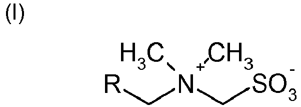 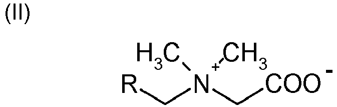 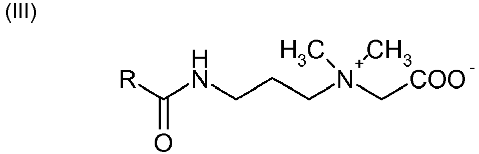 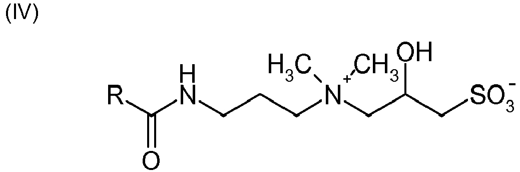 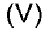 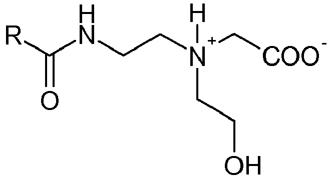 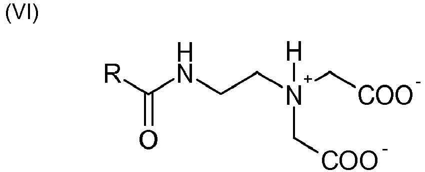 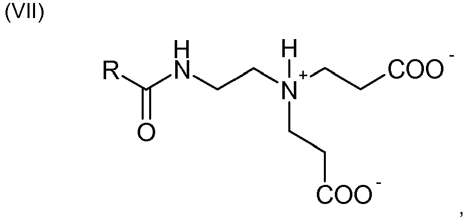
- mindestens ein auf probiotischen Mikroorganismen-basierendes Ferment, wobei das Ferment auf grampositiven Milchsäurebakterien (Lactobacillales) der Gattung *Lactococcus* und/oder *Lactobacillus* basiert.

Innerhalb dieser Ausführungsform ist es besonders bevorzugt, wenn die erfindungsgemäßen Reinigungsmittel - bezogen auf ihr Gesamtgewicht -
- 0,25 bis 12,5 Gew.-% mindestens eines Natrium-, Magnesium und/oder Triethanolaminsalzes eines linearen oder verzweigten Lauryl-, Tridecyl- und/oder Myristylsulfates, das einen mittleren Ethoxylierungsgrad von 2 bis 4 aufweist,
- 0,1 bis 8 Gew.-% mindestens eines amphoteren und/oder zwitterionischen Tensids einer der zuvor genannten Formeln (III), (V), (VI) oder (VII) und
- mindestens ein auf probiotischen Mikroorganismen-basierendes Ferment enthalten, wobei das Ferment auf den grampositiven Milchsäurebakterien *Lactococcus lactis* (subsp. *lactis, cremoris* und/oder *hordniae), Lactococcus garvieae, Lactococcus plantarum, Lactococcus raffinolactis* und/oder *Lactococcus piscium* basiert.

Innerhalb dieser Ausführungsform ist es insbesondere bevorzugt, wenn die erfindungsgemäßen Reinigungsmittel - bezogen auf ihr Gesamtgewicht -
- 1 bis 8 Gew.-% Natriumlaurylethersulfat, das einen mittleren Ethoxylierungsgrad von 2 bis 4 aufweist,
- 0,5 bis 5 Gew.-% mindestens eines der unter den INCI-Bezeichnungen Cocamidopropylbetain und/oder Cocoamphodiacetate bekannten amphoteren und/oder zwitterionischen Tenside und
- mindestens ein unter der INCI-Bezeichnung "Lactococcus Ferment and Propylene Glycol" bekanntes Ferment enthalten.

Die erfindungsgemäßen Reinigungsmittel eignen sich insbesondere für die Reinigung sensibler, feuchter Hautpartien, die in besonderer Weise vor dem Befall schädlicher Keime geschützt werden müssen.

Das Wachstum einer Reihe pathogener Mikroorganismen auf der Haut (beispielsweise *Propionibacterium acnes*) kann durch einen aciden pH-Wert gehemmt werden. Gleichzeitig beeinflusst ein acider pH-Wert das Wachstum einer Vielzahl von Mikroorganismen der natürlichen Hautflora.

Bevorzugte erfindungsgemäße Reinigungsmittel weisen daher einen pH-Wert im aciden Bereich von etwa 3 bis 5,5 auf, mehr bevorzugt von etwa 3,2 bis 5 und insbesondere von 3,5 bis 4,8 auf.

Neben den zuvor genannten Wirkstoffen können erfindungsgemäße Reinigungsmittel noch eine Reihe weiterer Wirkstoffe enthalten, die ihnen vorteilhafte Eigenschaften verleihen.

Zu den bevorzugten fakultativen Wirkstoffen, die in den erfindungsgemäßen Reinigungsmitteln eingesetzt werden können, zählen beispielsweise nichtionische Tenside und/oder nichtionische Emulgatoren sowie weitere - von a) verschiedene - anionische Tenside.

Besonders sensible erfindungsgemäße Reinigungsmittel, die die Reizung der Haut/Schleimhaut minimieren, können neben den Tensiden a) und b) zusätzlich mindestens ein mildes nichtionisches Tensid und/oder einen milden nichtionischen Emulgator enthalten.

Bevorzugte nichtionische Tenside und/oder Emulgatoren, die in den erfindungsgemäßen Reinigungsmitteln eingesetzt werden können, wirken bevorzugt schaumstabilisierend und verbessern die dermatologischen Eigenschaften des (der) anionischen Tensids(e).

Geeignete nichtionische Tenside und/oder Emulgatoren können beispielsweise ausgewählt sein aus
- Aminoxiden der nachfolgenden Formel (VIII), 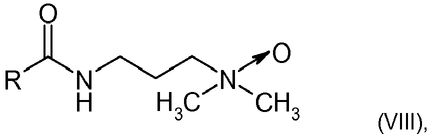 in denen R für einen geradkettigen oder verzweigten, gesättigten oder ein- oder mehrfach ungesättigten Alkyl- oder Alkenylrest mit 7 bis 17 Kohlenstoffatomen steht,
- Anlagerungsprodukten von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- Anlagerungsprodukten von 5 bis 60 Mol Ethylenoxid an Rizinusöl und/oder gehärtetes Rizinusöl,
- Zuckerfettsäureestern und Anlagerungsprodukten von Ethylenoxid an Zuckerfettsäureestern,
- Fettsäurealkanolamiden,
- Anlagerungsprodukten von Ethylenoxid an Fettamine und/oder
- Alkylpolyglucosiden.

Bevorzugte nichtionische Tenside/Emulgatoren sind Aminoxide der zuvor genannten Formel (VIII) und/oder Alkyloligoglucoside.

Besonders bevorzugt sind Alkyloligoglucoside auf der Basis von gehärtetem C_{12/14}-Kokosalkohol mit einem DP von 1-3, wie sie beispielsweise unter der INCI-Bezeichnung "Coco-Glucoside" im Handel erhältlich sind, sowie Aminoxide der zuvor genannten Formel (VIII), in der R für einen aus natürlichem Kokosfett erhältlichen Rest steht.

Insbesondere bevorzugt ist ein unter der INCI-Bezeichnung Cocamidopropylaminoxid bekanntes und im Handel von verschiedenen Anbietern erhältliches Tensid der zuvor genannten Formel (VIII).

Das oder die nichtionischen Tenside und/oder Emulgatoren kann (können) in den erfindungsgemäßen Reinigungsmitteln - bezogen auf deren Gesamtgewicht - bevorzugt in Mengen von 0,01 bis 10 Gew.-%, besonders bevorzugt von 0,05 bis 5 Gew.-% und insbesondere von 0,1 bis 2 Gew.-% eingesetzt werden.

Zu den geeigneten weiteren - von a) verschiedenen - anionischen Tensiden zählen beispielsweise:
- Ethercarbonsäuren der Formel R-O-(CH₂-CH₂O)ₓ-CH₂-COOH, in der R eine lineare oder verzweigte, gesättigte oder ungesättigte Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylglutamate mit 8 bis 24 C-Atomen in der Acylgruppe,
- Acylisethionate mit 8 bis 24 C-Atomen in der Acylgruppe,
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen, und/oder
- Alpha-Olefinsulfonate mit 8 bis 24 C-Atomen.

Die Hinzufügung zuvor genannter, fakultativer Tenside sollte die Milde der erfindungsgemäßen Reinigungsmittel nicht negativ beeinträchtigen.

Daher ist es von Vorteil, wenn erfindungsgemäße Reinigungsmittel, die neben den Tensiden a) und b) weitere nichtionische und/oder von a) verschiedene anionische Tenside enthalten, einen Gesamttensidgehalt von maximal 15 Gew.-%, bevorzugt von maximal 12,5 Gew.-% und insbesondere von maximal 10 Gew.-% aufweisen, wobei sich die Mengenangaben auf das Gesamtgewicht der Reinigungsmittel beziehen.

Zur weiteren Steigerung der milden und/oder pflegenden Eigenschaften der erfindungsgemäßen Reinigungsmittel ist es von Vorteil, wenn diese weiterhin mindestens einen Hautpflegestoff aus der folgenden Gruppe der:
- Vitamine, Vitaminderivate und/oder Vitaminvorstufen,
- Pflanzenextrakte,
- Pflanzenmilche,
- Hautfeuchthaltemittel und/oder
- Hautberuhigenden Wirkstoffe enthalten.

Unter geeigneten Vitaminen sind bevorzugt die folgenden Vitamine, Provitamine und Vitaminvorstufen sowie deren Derivate zu verstehen:
● Vitamin A: zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht.
● Vitamin B: zur Vitamin B-Gruppe oder zu dem Vitamin B-Komplex gehören u. a.
   ➢ Vitamin B₁ (Thiamin)
   ➢ Vitamin B₂ (Riboflavin)
   ➢ Vitamin B₃. Unter dieser Bezeichnung werden häufig die Verbindungen Nicotinsäure und Nicotinsäureamid (Niacinamid) geführt.
   ➢ Vitamin B₅ (Pantothensäure und Panthenol). Im Rahmen dieser Gruppe wird bevorzugt das Panthenol eingesetzt. Einsetzbare Derivate des Panthenols sind insbesondere die Ester und Ether des Panthenols, Pantolacton sowie kationisch derivatisierte Panthenole. Einzelne Vertreter sind beispielsweise das Panthenoltriacetat, der Panthenolmonoethylether und dessen Monoacetat sowie kationische Panthenolderivate.
   ➢ Vitamin B₆ (Pyridoxin sowie Pyridoxamin und Pyridoxal).
● Vitamin C (Ascorbinsäure): die Verwendung in Form des Palmitinsäureesters, der Glucoside oder Phosphate kann bevorzugt sein. Die Verwendung in Kombination mit Tocopherolen kann ebenfalls bevorzugt sein.
● Vitamin E (Tocopherole, insbesondere α-Tocopherol).
● Vitamin F: unter dem Begriff "Vitamin F" werden üblicherweise essentielle Fettsäuren, insbesondere Linolsäure, Linolensäure und Arachidonsäure, verstanden.
● Vitamin H: Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat.

Die erfindungsgemäßen Reinigungsmittel können bevorzugt Vitamine, Provitamine und Vitaminvorstufen aus den Gruppen A, B, E und H enthalten.

Insbesondere bevorzugt sind Nicotinsäureamid, Biotin, Pantolacton und/oder Panthenol.

Vitamine, Vitaminderivate und/oder Vitaminvorstufen können in den Reinigungsmitteln (bezogen auf deren Gesamtgewicht) bevorzugt in einer Menge von 0,001 bis 2 Gew.-%, besonders bevorzugt von 0,005 bis 1 Gew.-% und insbesondere von 0,01 bis 0,5 Gew.-% eingesetzt werden.

Unter geeigneten Pflanzenextrakten sind Extrakte zu verstehen, die aus allen Teilen einer Pflanze hergestellt werden können.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Geeignet sind vor allem die Extrakte aus Grünem Tee, Eichenrinde, Brennessel, Hamamelis, Hopfen, Kamille, Klettenwurzel, Schachtelhalm, Weißdorn, Lindenblüten, Litschi, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Ginseng, Ingwerwurzel, Echinacea purpurea, Olea europea, Boerhavia Diffusa-Wurzeln, Foeniculum vulgaris und Apim graveolens.

Besonders bevorzugt für die Verwendung in den erfindungsgemäßen Reinigungsmitteln sind die Extrakte aus Grünem Tee, Brennessel, Hamamelis, Kamille, Thymian, Aloe Vera, Ginseng, Echinacea purpurea, Olea europea und/oder Boerhavia Diffusa-Wurzeln.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Die Pflanzenextrakte können in den erfindungsgemäßen Mitteln (bezogen auf ihr Gesamtgewicht) bevorzugt in einer Menge von 0,01 bis 10 Gew.-%, mehr bevorzugt von 0,05 bis 7,5 Gew.-% und insbesondere von 0,1 bis 5 Gew.-% eingesetzt werden.

Unter geeigneten Pflanzenmilchen werden bevorzugt milchähnliche Flüssigkeiten verstanden, die aus rein pflanzlichen Quellen, wie beispielsweise aus Reis, Sojabohnen, Kokosnüssen, Mandeln, Quinoa, Baumwolle, Rosen und/oder Aloe Vera, erhalten werden können.

Besonders bevorzugte Pflanzenmilche, die in den erfindungsgemäßen Reinigungsmitteln eingesetzt werden können, sind Pflanzenmilche, die aus Aloe Vera, Kokosnüssen und/oder Sojabohnen erhältlich sind.

Entsprechende Handelsprodukte sind beispielsweise unter den Bezeichnungen Botanical Milk^{®} oder Extrapone Milk^{®} von der Firmen Arch und Symrise erhältlich.

Die Pflanzenmilch(e) kann (können) in den erfindungsgemäßen Reinigungsmitteln bevorzugt in Mengen von 0,001 bis 5 Gew.-%, besonders bevorzugt von 0,005 bis 4 Gew.-% und insbesondere von 0,01 bis 3 Gew.-% eingesetzt werden, wobei sich die Mengenangaben auf das Gesamtgewicht der Reinigungsmittel beziehen.

Geeignete Hautfeuchthaltende Wirkstoffe werden auch als Moisturizing-Komponenten bezeichnet. Darunter werden üblicherweise kosmetische Wirkstoffe verstanden, die der Austrocknung der Hornschicht der Epidermis während der Reinigung entgegenwirken, und damit zur erhöhten Geschmeidigkeit und Elastizität der Haut beitragen können.

Beispiele für geeignete Moisturizing-Komponenten sind Kollagenpräparate, ethoxylierte Glycosederivate, Glycerin, Sorbitol, Milchsäure, Aloe vera, Aminosäuren sowie deren Salze und Ester, insbesondere Chitosoniumpyrrolidoncarbonsäuresalze, Proteine, Proteinhydrolysate, Pyrrolidoncarbonsäure, weitere Bestandteile des "Natural Moisturizing Factor", der aus 40% freien Aminosäuren, 12% Pyrrolidoncarbonsäure, 12% Lactaten, 7% Harnstoff, 1,5% Harnsäure sowie D-Glucosamin, Kreatinin und verschiedenen Salzen besteht, Desoxyzucker, besonders bevorzugt Rhamnose und Fucose, Polysaccharide, die mindestens einen Desoxyzucker-Baustein enthalten, besonders bevorzugt die Handelsprodukte Fucogel^{®} (INCI-Bezeichnung Biosaccharide Gum-1) von Solabia, Rhamnosoft^{®} (INCI-Bezeichnung Biosaccharide Gum-2) von Solabia, Fucogenol^{®} (INCI-Bezeichnung Biosaccharide Gum-3) von Solabia und Glycofilm^{®} (INCI-Bezeichnung Biosaccharide Gum-4) von Solabia, weiterhin Mischungen der vorgenannten, mindestens einen Desoxyzucker-Baustein enthaltenden Polysaccharide, beispielsweise der Mischung aus Biosaccharide Gum-2 und Biosaccharide Gum-3, erhältlich als Handelsprodukt Elastinol plus^{®} von Solabia, weiterhin Harnstoff, N,N'-Bis(2-hydroxyethyl)harnstoff, Betain (Me₃N⁺-CH₂-COO⁻), Glycosaminoglycan, besonders bevorzugt Hyaluronsäure, Dextran, Dextransulfat, Chondroitin-4-sulfat und Chondroitin-6-sulfat, sowie beliebige Mischungen dieser Substanzen.

Der oder die Hautfeuchthaltende(n) Wirkstoff(e) kann (können) in den erfindungsgemäßen Reinigungsmitteln bevorzugt in einer Menge von 0,001 bis 10 Gew.-%, mehr bevorzugt von 0,01 bis 5 Gew.-% und besonders bevorzugt von 0,05 bis 3 Gew.-% enthalten sein. Die Mengenangaben beziehen sich dabei auf das Gesamtgewicht der Reinigungsmittel.

Unter geeigneten Hautberuhigenden Wirkstoffen sind beispielsweise Allantoin, α-Bisabolol, α-Liponsäure, Extrakte aus Centella asiatica, beispielsweise erhältlich unter der Bezeichnung Madecassicoside von DSM, Glycyrrethinsäure, die besonders bevorzugt in Liposomen verkapselt vorliegt und in dieser Form z. B. unter dem Handelsnamen Calmsphere von Soliance erhältlich ist, Mischungen aus Getreidewachsen, Extrakte aus Schibutter und Argania Spinosa-Öl mit der INCI-Bezeichnung "Spent grain wax and Butyrospermum Parkii (shea butter) extract and Argania Spinosa Kernel Oil", wie sie z. B. unter der Handelsbezeichnung Stimu-Tex AS von der Firma Pentapharm erhältlich sind, Extrakte aus Vanilla Tahitensis, wie sie z. B. unter der Handelsbezeichnung Vanirea (INCI : Vanilla Tahitensis Fruit Extract) von der Firma Solabia erhältlich sind, Alginhydrolysate, wie sie z. B. unter der Handelsbezeichnung Phycosaccharide, insbesondere Phycosaccharide Al, von der Firma Codif erhältlich sind, Extrakte aus Bacopa Monniera, wie sie z. B. unter der Handelsbezeichnung Bacocalmine von der Firma Sederma erhältlich sind, Extrakte aus der Rooibos-Pflanze, wie sie z. B. unter dem Handelsnamen Rooibos Herbasec MPE von der Firma Cosmetochem erhältlich sind, Hefeextrakte, besonders bevorzugt das Handelsprodukt Drieline (INCI-Bezeichnung "Sorbitol, Yeast Extract"), erhältlich von der Firma Lanatech, physiologisch verträgliche Salze von Sterolsulfaten, wie sie z. B. unter der Handelsbezeichnung Phytocohesine (INCI: Sodium Beta-Sitosterylsulfate) von der Firma Vincience erhältlich sind, Aminodicarbonsäuren mit einer C-Kettenlänge von 3 - 6 Kohlenstoffatomen sowie deren physiologisch verträgliche Salze, bevorzugt Aminomalonsäure, Aminobernsteinsäure (= Asparaginsäure), Aminoglutarsäure und Aminoadipinsäure sowie deren physiologisch verträgliche Salze wie Kaliumaspartat und Magnesiumaspartat, sowie beliebige Mischungen dieser Substanzen.

Der oder die Hautberuhigende(n) Wirkstoff(e) kann (können) in den erfindungsgemäßen Reinigungsmitteln bevorzugt in einer Menge von 0,001 - 5 Gew.-%, besonders bevorzugt von 0,005 - 3 Gew.-% und insbesondere von 0,01 - 2 Gew.-% enthalten sein, wobei sich die Mengenangaben auf das Gesamtgewicht der Reinigungsmittel beziehen.

In einer zweiten bevorzugten Ausführungsform enthalten erfindungsgemäße Reinigungsmittel - bezogen auf deren Gesamtgewicht -
- 0,1 bis 15 Gew.-% mindestens eines geradkettigen oder verzweigten Alkylethersulfats, das einen Alkylrest mit 8 bis 18 und insbesondere mit 10 bis 16 C-Atomen, sowie 1 bis 6 und insbesondere 2 bis 4 Ethylenoxideinheiten aufweist,
- 0,05 bis 10 Gew.-% mindestens eines amphoteren und/oder zwitterionischen Tensids b), das ausgewählt sein kann aus einer oder mehreren Verbindungen der nachfolgenden Formeln (I) bis (VII), in denen der Rest R für einen geradkettigen oder verzweigten, gesättigten oder ein- oder mehrfach ungesättigten Alkyl- oder Alkenylrest mit 7 bis 23 Kohlenstoffatomen (Formeln (I) und (II)) oder für einen geradkettigen oder verzweigten, gesättigten oder ein- oder mehrfach ungesättigten Alkyl- oder Alkenylrest mit 8 bis 24 Kohlenstoffatomen (Formeln (III) bis (VII)) steht 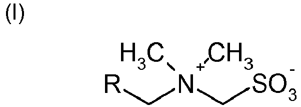 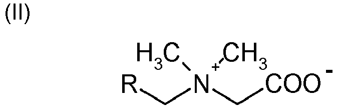 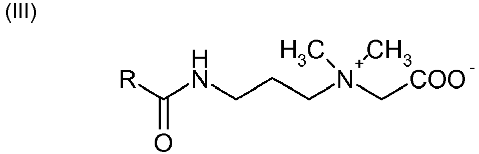 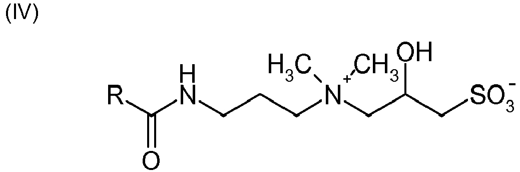 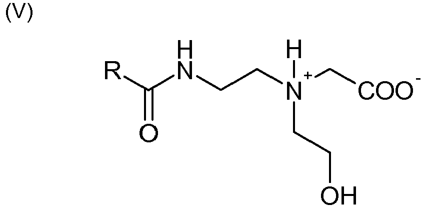 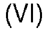 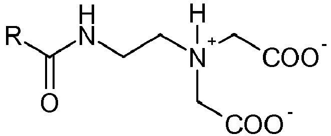 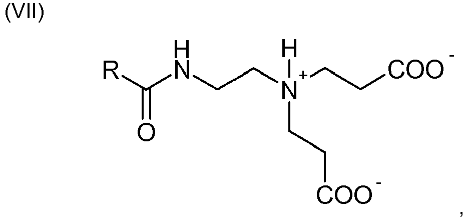
- mindestens ein auf probiotischen Mikroorganismen-basierendes Ferment, wobei das Ferment auf grampositiven Milchsäurebakterien (Lactobacillales) der Gattung *Lactococcus* und/oder *Lactobacillus* basiert und
- 0,01 bis 10 Gew.-% mindestens eines nichtionischen Tensids und/oder eines nichtionischen Emulgators, ausgewählt aus
- Aminoxiden der nachfolgenden Formel (VIII), 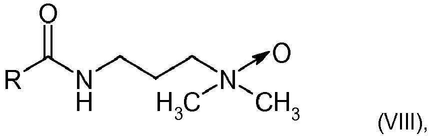 in denen R für einen geradkettigen oder verzweigten, gesättigten oder
   ein- oder mehrfach ungesättigten Alkyl- oder Alkenylrest mit 7 bis 17 Kohlenstoffatomen steht,
- Anlagerungsprodukten von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- Anlagerungsprodukten von 5 bis 60 Mol Ethylenoxid an Rizinusöl und/oder ge härtetes Rizinusöl,
- Zuckerfettsäureestern und Anlagerungsprodukten von Ethylenoxid an Zuckerfettsäureestern,
- Fettsäurealkanolamiden,
- Anlagerungsprodukten von Ethylenoxid an Fettamine und/oder
- Alkylpolyglucosiden.

Innerhalb dieser Ausführungsform ist es besonders bevorzugt, wenn die erfindungsgemäßen Reinigungsmittel - bezogen auf ihr Gesamtgewicht -
- 0,25 bis 12,5 Gew.-% mindestens eines Natrium-, Magnesium und/oder Triethanolaminsalzes eines linearen oder verzweigten Lauryl-, Tridecyl- und/oder Myristylsulfates, das einen mittleren Ethoxylierungsgrad von 2 bis 4 aufweist,
- 0,1 bis 8 Gew.-% mindestens eines amphoteren und/oder zwitterionischen Tensids einer der zuvor genannten Formeln (III), (V), (VI) oder (VII),
- mindestens ein auf probiotischen Mikroorganismen-basierendes Ferment, wobei das Ferment auf den grampositiven Milchsäurebakterien *Lactococcus lactis* (subsp. *lactis, cremoris* und/oder *hordniae*)*, Lactococcus garvieae, Lactococcus plantarum, Lactococcus raffinolactis* und/oder *Lactococcus piscium* basiert,
- 0,05 bis 5 Gew.-% mindestens eines Aminoxids der zuvor genannten Formel (VIII) und/oder eines Alkyloligoglucosids und/oder
- mindestens einen Hautpflegestoff, ausgewählt aus der folgenden Gruppe der
   ➢ Vitamine, Vitaminderivate und/oder Vitaminvorstufen,
   ➢ Pflanzenextrakte,
   ➢ Pflanzenmilche,
   ➢ Hautfeuchthaltemittel und/oder
   ➢ Hautberuhigenden Wirkstoffe enthalten.

Innerhalb dieser Ausführungsform ist es insbesondere bevorzugt, wenn die erfindungsgemäßen Reinigungsmittel - bezogen auf ihr Gesamtgewicht -
- 1 bis 8 Gew.-% Natriumlaurylethersulfat, das einen mittleren Ethoxylierungsgrad von 2 bis 4 aufweist,
- 0,5 bis 5 Gew.-% mindestens eines der unter den INCI-Bezeichnungen Cocamidopropylbetain und/oder Cocoamphodiacetate bekannten amphoteren und/oder zwitterionischen Tenside,
- mindestens ein unter der INCI-Bezeichnung "Lactococcus Ferment and Propylene Glycol" bekanntes Ferment,
- 0,1 bis 2 Gew.-% mindestens eines unter der INCI-Bezeichnung Cocamidopropylaminoxid bekannten nichtionischen Tensids und/oder
- mindestens einen Hautpflegestoff enthalten, der ausgewählt ist aus
   ➢ Nicotinsäureamid, Biotin, Pantolacton und/oder Panthenol und/oder
   ➢ einem Pflanzenextrakt aus Grünem Tee, Brennessel, Hamamelis, Kamille, Thymian, Aloe Vera, Ginseng, Echinacea purpurea, Olea europea und/oder Boerhavia Diffusa-Wurzeln und/oder
   ➢ einer Pflanzenmilch, die aus Aloe Vera, Kokosnüssen und/oder Sojabohnen erhältlich ist, und/oder
   ➢ einem Hautfeuchthaltemittel, ausgewählt aus Glycerin, Sorbitol, Milchsäure, Aminosäuren sowie deren Salzen und Estern, Proteinhydrolysaten und/oder Hyalurobsäure, und/oder
   ➢ einem Hautberuhigenden Wirkstoff, ausgewählt aus Allantoin und/oder alpha-Bisabolol.

In einer weiteren Ausführungsform kann es bevorzugt sein, wenn die erfindungsgemäßen Reinigungsmittel zusätzlich mindestens eine weitere die Haut pflegende Komponente enthalten. Die pflegende(n) Komponente(n) kann (können) bevorzugt ausgewählt sein aus natürlichen, synthetischen und/oder mineralischen Ölkomponenten und/oder Rückfettungskomponenten sowie aus kationischen Polymeren.

Als natürliche (pflanzliche) Öle werden üblicherweise Triglyceride und Mischungen von Triglyceriden eingesetzt. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaoabutter und Shea-Butter.

Als mineralische Öle kommen insbesondere Mineralöle, Paraffin- und Isoparaffinöle sowie synthetische Kohlenwasserstoffe zum Einsatz. Ein Beispiel für einen einsetzbaren Kohlenwasserstoff ist beispielsweise das als Handelsprodukt erhältliche 1,3-Di-(2-ethylhexyl)-cyclohexan (Cetiol^{®} S).

Als synthetische Öle können Dialkylether, beispielsweise Di-n-octylether, Fettsäuren, Fettalkohole sowie natürliche und synthetische Wachse eingesetzt werden, welche sowohl in fester Form als auch flüssig in wässriger Dispersion vorliegen können.

Beispiele für geeignete Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.

Bevorzugt sind üblicherweise die Fettsäureschnitte, welche aus Cocosöl oder Palmöl erhältlich sind; insbesondere bevorzugt ist in der Regel der Einsatz von Stearinsäure.

Geeignete Fettalkohole sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole.

Als natürliche oder synthetische Wachse können eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP.

Weitere geeignete Fettstoffe sind beispielsweise
- Esteröle. Unter Esterölen sind zu verstehen die Ester von C₆ - C₃₀ - Fettsäuren mit C₂ - C₃₀-Fettalkoholen. Bevorzugt sind beispielsweise Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen,
- Glycerincarbonat oder Dicaprylylcarbonat (Cetiol^{®} CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuis^{®} 90-018, Monomuls^{®} 90-L12, Cetiol^{®} HE oder Cutina^{®} MD.

Besonders bevorzugte Ölkomponenten, die in den erfindungsgemäßen Reinigungsmitteln eingesetzt werden können, sind ausgewählt aus zuvor genannten natürlichen Ölen, Esterölen und/oder ethoxylierten oder nicht ethoxylierten Mono,- Di- und Trifettsäureestern von gesättigten und/oder ungesättigten linearen und/oder verzweigten Fettsäuren mit Glycerin.

Die zuvor genannten Ölkomponenten dienen bevorzugt auch als Rückfettungsmittel für die Haut.

Die zuvor genannten, bevorzugten Ölkomponenten - insbesondere die (gegebenenfalls ethoxylierten) Mono-, Di- und/oder Triester des Glycerins mit mindestens einer C₈-C₂₄-Fettsäure - können in den erfindungsgemäßen Reinigungsmitteln bevorzugt in Mengen von 0,01 bis 5 Gew.-%, mehr bevorzugt von 0,025 bis 4 Gew.-%, besonders bevorzugt von 0,05 bis 3 Gew.-% und insbesondere von 0,1 bis 2 Gew.-% eingesetzt werden, wobei sich die Mengenangaben auf das Gesamtgewicht der Reinigungsmittel beziehen.

Geeignete kationische Polymere sind beispielsweise:
- quaternisierte Cellulose-Derivate, wie sie unter den Bezeichnungen Celquat^{®} und Polymer JR^{®} im Handel erhältlich sind,
- hydrophob modifizierte Cellulosederivate, beispielsweise die unter dem Handelsnamen SoftCat^{®} vertriebenen kationischen Polymere,
- kationische Alkylpolyglycoside,
- kationiserter Honig, beispielsweise das Handelsprodukt Honeyquat^{®} 50,
- kationische Guar-Derivate, wie insbesondere die unter den Handelsnamen Cosmedia^{®}Guar N-Hance^{®} und Jaguar^{®} vertriebenen Produkte,
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Be zeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
   sowie die unter den Bezeichnungen
- Polyquaternium 2, Polyquaternium 17, Polyquaternium 18, Polyquaternium-24, Polyquaternium 27, Polyquaternium-32, Polyquaternium-37, Polyquaternium 74 und Polyquaternium 89 bekannten Polymere.

Bevorzugte kationische Polymere sind quaternisierte Cellulosepolymere, kationische Guarderivate und/oder kationische Polymere auf Acrylsäure(derivat)basis, die besonders bevorzugt ausgewählt sind aus den unter den INCI-Bezeichnungen bekannten Polymeren Guar Hydroxypropyltrimonium Chloride, Polyquaternium-6, Polyquaternium-7, Polyquaternium-10, Polyquaternium-37 und/oder Polyquaternium-67.

Das oder die kationische(n) Polymer(e), kann (können) in den erfindungsgemäßen Reinigungsmitteln (bezogen auf ihr Gesamtgewicht) bevorzugt in einer Menge 0,01 bis 5 Gew.-%, mehr bevorzugt von 0,025 bis 4 Gew.-%, besonders bevorzugt von 0,05 bis 3 Gew.-% und insbesondere von 0,1 bis 2 Gew.-% eingesetzt werden.

Weitere Wirk-, Hilfs- und Zusatzstoffe, die in den erfindungsgemäßen Reinigungsmitteln eingesetzt werden können, sind beispielsweise:
- Farbstoffe zum Anfärben des Mittels,
- Substanzen zur Einstellung des pH-Wertes, beispielsweise alpha- und beta-Hydroxycarbonsäuren wie Citronensäure, Äpfelsäure, Glycolsäure, sowie NaOH,
- Komplexbildner wie EDTA, NTA, β-Alanindiessigsäure und Phosphonsäuren,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Treibmittel wie Propan-Butan-Gemische, N₂O, Dimethylether, CO₂ und Luft,
- Antioxidantien,
- Parfums,
- Verdickungsmittel wie beispielsweise Assoziativverdickundsmittel und/oder Elektrolyte wie Natriumchlorid,
- Konservierungsmittel, wie beispielsweise Benzoesäure und/oder Salicylsäure bzw. deren physiologisch verträgliche Salze.

Erfindungsgemäß bevorzugte kosmetische Reinigungsmittel weisen bevorzugt eine Viskosität im Bereich von 500 bis 2,000 mPas, bevorzugt von 600 bis 1,800 mPas und insbesondere von 750 bis 1,750 mPas auf (jeweils gemessen mit einem Haake Rotationsviskosimeter VT550; 20°C; Meßeinrichtung MV; Spindel MV II; 8 UPM).

Zusammensetzungen dieser - vergleichsweise niedrigen - Viskosität lassen sich gut auf den zu reinigenden Hautpartien verteilen und nach der Reinigung besonders gut wieder von der Haut abspülen.

Die erfindungsgemäßen Reinigungsmittel weisen hervorragende Eigenschaften in der Anwendung auf der Haut auf.

Sie sind probiotisch, besonders mild und sehr gut haut- und schleimhautverträglich. Weiterhin hinterlassen sie nach dem Abspülvorgang ein frisches Hautgefühl, bekämpfen wirksam unangenehme Gerüche und bieten einen natürlichen Schutz für die Haut.

Die erfindungsgemäßen Reinigungsmittel können Hautirritationen vorbeugen und/oder verringern und eignen sich hervorragend für die Behandlung sensibler und/oder feuchter Hautpartien wie beispielsweise der Intimregion, der Achselhöhlen und/oder der Zehenzwischenräume.

Ein zweiter Gegenstand der Erfindung ist daher die Verwendung des erfindungsgemäßen Reinigungsmittels als Intimreinigungsmittel zur Stärkung der Hautbarriere, zur Erfrischung und/oder zur Bekämpfung unangenehmer Gerüche.

### Beispiele

Die folgende Tabelle enthält Beispiele für erfindungsgemäße Reinigungszusammensetzungen (Intimwaschlotionen).

Die Mengenangaben in der Tabelle beziehen sich - sofern nicht anders angegeben - auf Gew.-%.

| | **1** | **2** | **3** | **4** | **5** |
|---|---|---|---|---|---|
| Natriumlaurethsulfat (2EO, 70%) | 10 | 10 | 9 | 12 | 11 |
| Disodium Cocoamphodiacetate (40%) | 4 | 4 | 4 | - | - |
| Cocamidopropylbetain (40%) | - | - | - | 5 | 3 |
| Technobion^{®1} BL LS 8407 | 2 | 1,5 | 2 | 3 | 1 |
| Aminoxid^{®2} WS 35 | 2 | 2 | 1,5 | 3 | - |
| Coco Glucoside | - | - | - | - | 1 |
| Milchsäure (80%) | 0,2 | 0,2 | 0,2 | 0,3 | - |
| Aloe Milk^{®3} | - | 0,1 | - | - | - |
| Glycerin | - | 3 | 1 | - | 1 |
| Alpha-Bisabolol | - | - | - | 0,05 | - |
| Thymianextrakt | - | - | - | - | 0,1 |
| Polyquaternium-10 | - | - | - | - | 0,3 |
| Mandelöl, süß | - | - | 0,1 | - | - |
| Olivenöl | - | - | - | 0,1 | - |
| Parfum | q.s. | q.s. | q.s. | q.s. | q.s. |
| Säuerungsmittel, Konservierungsmittel, Verdickungsmittel | q.s. | q.s. | q.s. | q.s. | q.s. |
| Wasser | ad 100 | ad 100 | 1 | 1 | |

In den Reinigungsmitteln der Beispiele 1 bis 5 wurden die folgenden Handelsprodukte eingesetzt:
1 INCI-Bezeichnung: Lactococcus Ferment and Propylene Glycol; Cognis
2 INCI-Bezeichnung: Cocamidopropylamine Oxide; 35% AS; Evonik Goldschmidt
3 INCI-Bezeichnung: Water, Amygdalus Dulcis Oil, Aloe Barbadensis Leaf Extract, Ceteareth-6, Stearyl Alcohol, Phenoxyethanol, Methylparaben, Ethylparaben, Propylparaben, Butylparaben, Isobutylparaben; Cosmetochem

## Patentansprüche

1. Kosmetisches Reinigungsmittel, enthaltend in einem geeigneten Träger
a) mindestens ein Alkylsulfat- und/oder ein Alkylpolyglykolethersulfatsalz,
b) mindestens ein amphoteres und/oder zwitterionisches Tensid und
c) mindestens ein auf probiotischen Mikroorganismen-basierendes Ferment, wobei die Mikroorganismen aus Milch isoliert wurden.

2. Kosmetisches Reinigungsmittel nach Anspruch 1, **dadurch gekennzeichnet, dass** es - bezogen auf sein Gesamtgewicht - 0,1 bis 15 Gew.-%, bevorzugt 0,25 bis 12,5 Gew.-%, besonders bevorzugt 0,5 bis 10 Gew.-% und insbesondere 1 bis 8 Gew.-% mindestens eines Alkylsulfat- und/oder Alkylpolyglykolethersulfatsalzes und/oder 0,05 bis 10 Gew.-%, bevorzugt 0,1 bis 8 Gew.-%, besonders bevorzugt 0,25 bis 6,5 Gew.-% und insbesondere 0,5 bis 5 Gew.- % mindestens eines amphoteren und/oder zwitterionischen Tensids enthält.

3. Kosmetisches Reinigungsmittel nach einem der Ansprüche 1 oder 2, **dadurch gekennzeichnet, dass** es als amphoteres und/oder zwitterionisches Tensid b) mindestens eine Verbindung der nachfolgenden Formeln (I) bis (VII) enthält, in denen der Rest R für einen geradkettigen oder verzweigten, gesättigten oder ein- oder mehrfach ungesättigten Alkyl- oder Alkenylrest mit 7 bis 23 Kohlenstoffatomen (Formeln (I) und (II)) oder für einen geradkettigen oder verzweigten, gesättigten oder ein- oder mehrfach ungesättigten Alkyl- oder Alkenylrest mit 8 bis 24 Kohlenstoffatomen (Formeln (III) bis (VII)) steht,

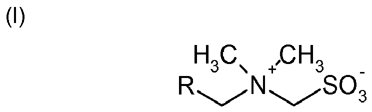

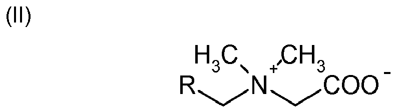

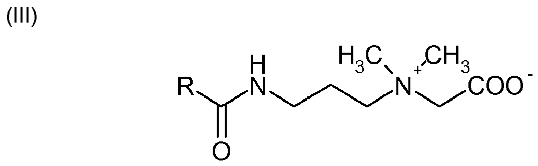

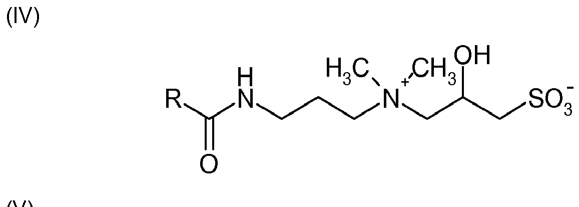

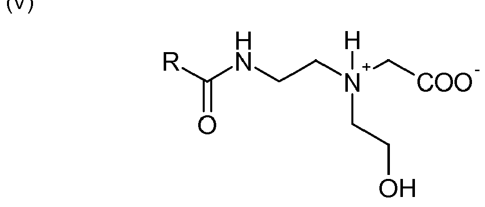

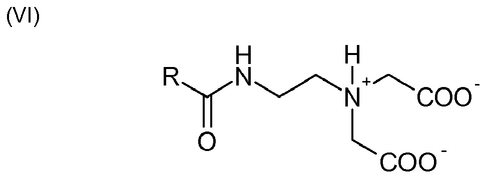

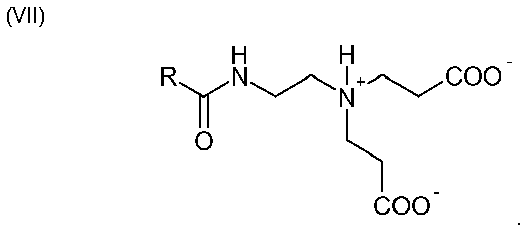

4. Kosmetisches Reinigungsmittel nach einem der Ansprüche 1 bis 3, **dadurch gekennzeichnet, dass** das Ferment c) auf grampositiven Milchsäurebakterien (Lactobacillales) der Gattung *Lactococcus* und/oder *Lactobacillus* basiert.

5. Kosmetisches Reinigungsmittel nach Anspruch 4, **dadurch gekennzeichnet, dass** die Milchsäurebakterien ausgewählt sind aus *Lactococcus lactis* (subsp. *lactis, cremoris* und/oder *hordniae), Lactococcus garvieae, Lactococcus plantarum, Lactococcus raffinolactis* und/oder *Lactococcus piscium.*

6. Kosmetisches Reinigungsmittel nach einem der Ansprüche 1 bis 5, **dadurch gekennzeichnet, dass** es als Komponente c) ein unter der INCI-Bezeichnung "Lactococcus Ferment (and) Propylene Glycol" bekanntes Ferment enthält.

7. Kosmetisches Reinigungsmittel nach einem der Ansprüche 1 bis 6, **dadurch gekennzeichnet, dass** es einen pH-Wert im Bereich von 3 bis 5,5 auf, bevorzugt von etwa 3,2 bis 5 und insbesondere von 3,5 bis 4,8 aufweist.

8. Kosmetisches Reinigungsmittel nach einem der Ansprüche 1 bis 7, **dadurch gekennzeichnet, dass** es - bezogen auf sein Gesamtgewicht - zusätzlich 0,01 bis 10 Gew.-% eines nichtionischen Tensids und/oder eines nichtionischen Emulgators enthält.

9. Kosmetisches Reinigungsmittel nach einem der Ansprüche 1 bis 8, **dadurch gekennzeichnet, dass** es mindestens einen Stoff aus der Gruppe der
- Pflanzenextrakte,
- Pflanzenmilche,
- Vitamine, Provitamine und Vitaminvorstufen,
- Hautfeuchthaltemittel und/oder
- Hautberuhigenden Wirkstoffe enthält.

10. Verwendung eines Reinigungsmittels nach einem der Ansprüche 1 bis 9 als Intimreinigungsmittel zur Stärkung der Hautbarriere, zur Erfrischung und/oder zur Bekämpfung unangenehmer Gerüche.
